# EUROPEAN PATENT APPLICATION

(11) **EP 2 174 940 A1**
(43) Date of publication of application: **14.04.2010**
(21) Application number: 08790802.6
(22) Date of filing: 02.07.2008
(51) Int. Cl.: C07D 471/14, C07B 57/00, C07B 63/00, A61K 31/55, A61P 25/20

(54) **PROCESS FOR PRODUCTION OF OPTICALLY ACTIVE MIRTAZAPINE**

(30) Priority: 10.07.2007 JP 2007180645
(71) Applicant: Sumitomo Chemical Company, Limited, Tokyo 104-8260 (JP)
(72) Inventor: WANG, Weiqi, Toyonaka-shi Osaka 560-0021 (JP); IKEMOTO, Tetsuya, Toyonaka-shi Osaka 561-0874 (JP)
(74) Representative: Vossius & Partner
(86) International application number: PCT/JP2008/061982
(87) International publication number: WO 2009/008303

(57) **Abstract**

The invention provides a process for efficiently producing optically active mirtazapine. Specifically, a process for production of optically active mirtazapine wherein an RS mixture of mirtazapine is optically resolved with optically active tartaric acid in the presence of a solvent.

More specifically, a process for production of optically active mirtazapine, **characterized by** making an RS mixture of mirtazapine contact with optically active tartaric acid in the presence of a solvent, and preferentially crystallizing the optically active mirtazapine salt.

The solvent is preferably a mixed solvent consisting of a water-soluble organic solvent and water, and more preferably a mixed solvent consisting of a C1-3 alcohol and water.

## Description

### Technical Field

The present invention relates to a process for production of optically active mirtazapine.

### Background Art

Known processes for production of optically active mirtazapine include processes in which optically active O,O-dibenzoyltartaric acid is used for optical resolution of RS mixtures of mirtazapine (see Patent document 1). However, such processes give low yields despite the use of relatively expensive resolving agents, and they are therefore unsatisfactory from an industrial standpoint.
[Patent document 1] Japanese Unexamined Patent Publication SHO No. 51-122093

### Disclosure of the Invention

### Problems to be Solved by the Invention

With this in mind, the present inventors diligently researched methods for efficiently producing optically active mirtazapine by optical resolution of RS mixtures of mirtazapine, and as a result they have completed this invention upon finding that optically active mirtazapine can be obtained efficiently by using inexpensive optically active tartaric acid as the resolving agent.

### Means for Solving the Problems

Specifically, the present invention provides a process for producing optically active mirtazapine wherein an RS mixture of mirtazapine is optically resolved with optically active tartaric acid in the presence of a solvent.

### Effect of the Invention

According to the invention, it is possible to accomplish efficient optical resolution of RS mixtures of mirtazapine. Furthermore, the process is industrially advantageous from a cost standpoint since the resolving agent is inexpensive.

### Brief Description of the Drawings

Fig. 1 is a 1H-NMR spectrum chart for the L-tartaric acid salt of S-mirtazapine (Example 1).
Fig. 2 is a 1H-NMR spectrum chart for S-mirtazapine (Reference Example 1).
Fig. 3 shows the XRD data for S-mirtazapine (Reference Example 1).
Fig. 4 is a 1H-NMR spectrum chart for S-mirtazapine (Reference Example 2).
Fig. 5 shows the XRD data for S-mirtazapine (Reference Example 2).
Fig. 6 is a 1H-NMR spectrum chart for the D-tartaric acid salt of R-mirtazapine (Example 2).

### Best Mode for Carrying Out the Invention

The invention will now be explained in detail.

Mirtazapine is a compound represented by the following formula, and it exists as two isomers, the R-form and S-form. 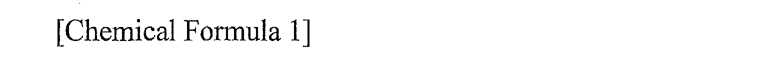 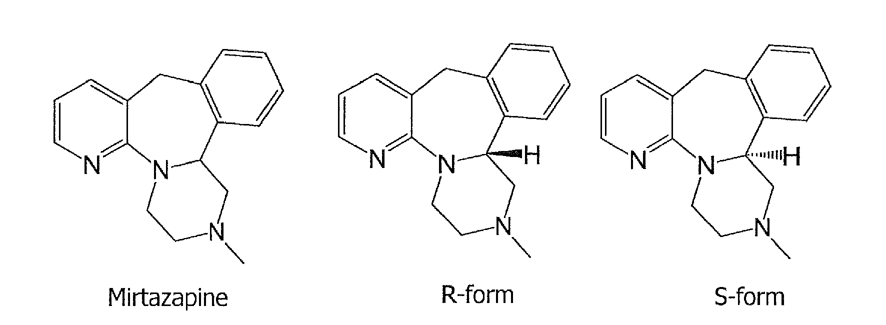 For the purpose of the invention, an RS mixture of mirtazapine is a mixture consisting of the R-form and S-form of mirtazapine in any proportion, and it may be a completely racemic mixture or it may contain either isomer in excess.

The optically active tartaric acid used may be a commercially available product in most cases. Preferred from the viewpoint of cost is L-tartaric acid.

The amount of optically active tartaric acid used will usually be in the range of 0.5 to 1.5-fold moles and preferably 0.8 to 1.2-fold moles with respect to the RS mixture of mirtazapine.

Examples of solvents include water-soluble organic solvents, water, and mixtures thereof. A mixed solvent consisting of a water-soluble organic solvent and water is preferred. The mixed solvent may be obtained by premixing the water-soluble organic solvent and water, or these may be mixed in the system during the optical resolution procedure. The mixing proportion of the water-soluble organic solvent and water is not particularly restricted, but it will usually be in the range of 1 to 100 times by weight and preferably 3 to 50 times by weight of the water-soluble organic solvent with respect to the water.

The water-soluble organic solvent is not particularly restricted so long as it is an organic solvent that can be homogeneously mixed with water in any proportion, and examples thereof include ether solvents such as tetrahydrofuran, 1,2-dimethoxymethane, 1,2-dimethoxyethane, diglyme, dioxane and 1,3-dioxolane; alcohol solvents such as methanol, ethanol, 1-propanol, 2-propanol, 2-methoxyethanol and 2-ethoxyethanol; ketone solvents such as acetone; sulfur-containing solvents such as dimethyl sulfoxide and sulfolane; nitrogen-containing solvents such as acetonitrile, N,N-dimethylformamide, N,N-dimethylacetamide, N,N-dimethylpropionamide, N-methylpyrrolidone, 1,3-dimethyl-2-imidazolidinone and 1,3-dimethyl-3,4,5,6-tetrahydro-2(1H)-pyridinone; lactone solvents such as γ-butyrolactone; and carbonic acid ester solvents such as dimethyl carbonate. Alcohol solvents are preferred, with methanol being more preferred.

The amount of solvent used will usually be in the range of 1 to 50 times by weight and preferably 5 to 20 times by weight with respect to the RS mixture of mirtazapine.

The optical resolution of the invention is carried out by making the RS mixture of mirtazapine contact with the optically active tartaric acid in the presence of the solvent. The order of combining the solvent, the RS mixture of mirtazapine and the optically active tartaric acid is not particularly restricted, and after being adjusted to a prescribed mixing temperature, an optically active mirtazapine salt will be preferentially deposited in most cases by cooling the mixture if necessary. Examples of specific modes include a mode in which the solvent, the RS mixture of mirtazapine and the optically active tartaric acid are combined all at once and the obtained mixture is adjusted to a prescribed mixing temperature to obtain a homogeneous solution, after which the solution is cooled for preferential deposition of an optically active mirtazapine salt; and a mode in which the RS mixture of mirtazapine is combined with a water-soluble organic solvent and the mixture is adjusted to a prescribed mixing temperature, after which optically active tartaric acid is added to and mixed with the mixture to form a homogeneous solution, water is added to and mixed therewith, and if necessary the mixture is cooled to preferentially deposit an optically active mirtazapine salt. In this method of optical resolution, the optically active mirtazapine salt may be used as seed crystals if necessary. The preferred mode is a mode in which the RS mixture of mirtazapine is combined with a water-soluble organic solvent and the mixture is adjusted to a prescribed mixing temperature, after which optically active tartaric acid is added to and mixed with the mixture to form a homogeneous solution, water is added thereto and mixed therewith to preferentially deposit an optically active mirtazapine salt, by cooling the mixture if necessary. Water may be further added during cooling or after cooling. When the optically active mirtazapine salt is deposited as crystals during the water-addition stage, the mixture may be cooled with the crystals or cooled after separating out the crystals, or it may be cooled after heating to dissolve the crystals, or the crystals may be separated out without cooling and subjected to the following post-treatment.

The mixing temperature is in the range of usually 35 to 100°C and preferably 35 to 65°C. The temperature upon completion of cooling is in the range of usually -10 to 30°C and preferably 5 to 30°C.

The mixture obtained by the optical resolution will normally contain the optically active mirtazapine salt crystals, and the optically active mirtazapine salt can be obtained by separating the crystals from the mixture. The separation may be accomplished by procedures such as filtration or decantation. If necessary, the solvent may be used for washing treatment of the optically active mirtazapine salt that has been obtained. The optically active mirtazapine salt may also be purified by ordinary purifying treatment such as recrystallization or column chromatography.

The liquid phase obtained by the separation may contain an abundant amount of optically active mirtazapine having the opposite configuration to that of the optically active mirtazapine salt that has been obtained, in which case the optically active mirtazapine may be isolated by ordinary post-treatment of the liquid phase, such as washing, concentration, distillation and crystallization.

The optically active mirtazapine salt obtained by the separation consists of optically active mirtazapine and optically active tartaric acid, and if L-tartaric acid is used as the optically active tartaric acid the optically active mirtazapine in the salt will normally be rich in the S-form, while if D-tartaric acid is used as the optically active tartaric acid the optically active mirtazapine in the salt will normally be rich in the R-form. The optically active mirtazapine salt may also be a hydrate. Making the optically active mirtazapine salt contact with a base will yield the corresponding optically active mirtazapine.

Examples of such bases include alkali metal hydroxides such as lithium hydroxide, sodium hydroxide and potassium hydroxide; alkali metal carbonates such as sodium carbonate and potassium carbonate; alkali metal hydrogencarbonates such as sodium hydrogencarbonate and potassium hydrogencarbonate; alkali metal alcoholates such as sodium methylate and sodium ethylate; and tertiary amines such as triethylamine, diisopropylethylamine and tributylamine.

Contact between the optically active mirtazapine salt and base can be accomplished in the presence of a solvent in most cases. Examples of such solvents include water; alcohol solvents such as methanol, ethanol and 2-propanol; and ether solvents such as tetrahydrofuran. The amount of base used will usually be in the range of 0.5 to 2-fold moles of the optically active tartaric acid in the optically active mirtazapine salt.

The contact temperature for the optically active mirtazapine salt and base will usually be in the range of 0 to 50°C. There are no particular restrictions on the order of mixing the optically active mirtazapine and base.

After the optically active mirtazapine salt and base have been contacted, the optically active mirtazapine may be isolated by ordinary post-treatment of the obtained mixture, such as liquid separation, concentration, distillation and crystallization. Specifically, there is a mode of liquid separation after combining with water and/or an organic solvent that is immiscible with water if necessary, and concentration of the obtained organic layer. The optically active mirtazapine may be further purified by ordinary purifying treatment such as recrystallization or column chromatography.

### Examples

The present invention will now be further explained by examples, with the understanding that the invention is not limited by the examples.

For each of the examples, the optical purity of mirtazapine was determined by the high-performance liquid chromatography area percentage. The analysis conditions were as follows.
<Analysis conditions>
Column: CHIRALCEL OD-H 4.6 mm × 250 mm, 40°C
Mobile phase: hexane/2-propanol (9/1)
Flow rate: 1 ml/min
Detector: UV (290 nm)
Retention time: 6.3 minutes (R-mirtazapine), 7.4 minutes (S-mirtazapine)

### Example 1

After combining 15.0 g (56.5 mmol, racemic mixture) of mirtazapine and 180 mL of methanol, the obtained mixture was heated to 50°C. To this there was added L-tartaric acid 8.65 g (57.6 mmol), and the mixture was agitated to obtain a homogeneous solution. Upon adding 18 mL of water dropwise thereto at the same temperature over a period of about 5 minutes, crystals were deposited. The obtained mixture was heated to 60°C to dissolve most of the crystals, and then cooled to 25°C over a period of about 22 hours. The crystals were separated from the obtained mixture by filtration, washed with 25 mL of methanol-water (10:1) and then dried under reduced pressure to obtain 12.5 g of crude crystals of the L-tartaric acid salt of S-mirtazapine as a white powder. After mixing 12.5 g of the crude crystals with 60 mL of methanol, the mixture was heated to 45°C and mixed, causing complete dissolution of the crystals. After adding 1.5 mL of water thereto and mixing, deposition of crystals was observed. The obtained mixture was heated to 64°C for dissolution of the crystals, and then cooled to 40°C over a period of about 1 hour, upon which crystals were deposited. After adding 4.5 mL of water over a period of 1 hour, the mixture was cooled to 25°C over a period of about 15 hours. The crystals were separated from the obtained mixture by filtration, washed with 15 mL of methanol-water (10:1) and then dried under reduced pressure to obtain 11.9 g of crude crystals of the L-tartaric acid salt of S-mirtazapine as a white powder. The optical purity of the S-mirtazapine in the obtained crystals was 100% ee. The water content of the crystals was 9.4 wt% as measured by the Karl Fischer method.

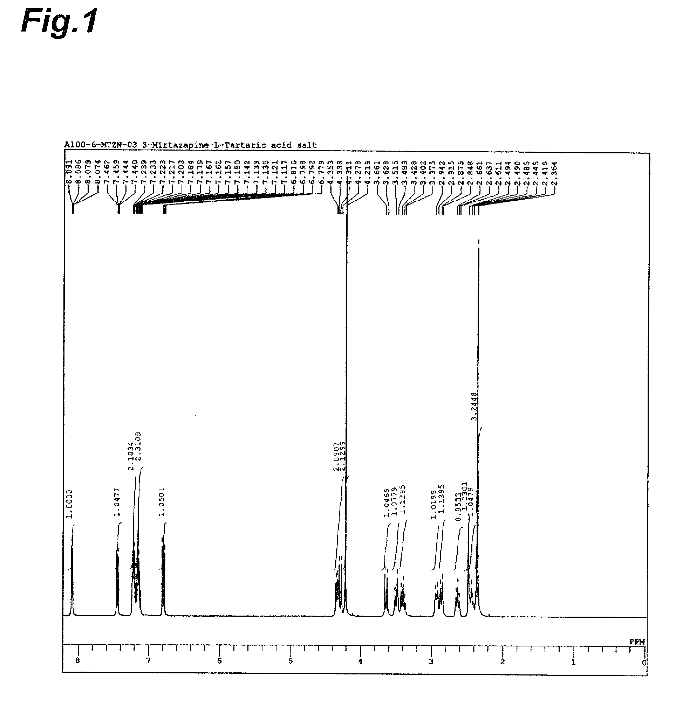

Fig. 1 shows a 1H-NMR spectrum chart for the L-tartaric acid salt of S-mirtazapine.
δ(ppm in DMSO-d6):2.34(1H,m), 2.37(3H,s), 2.51(1H,t-like, J = ca.11 Hz), 2.86(1H,dt J = 11,2 Hz), 2.96(1H,dd, J = 11,2 Hz), 3.42(1H,d, J = 13 Hz), 3.47(1H,m), 3.69(1H,m), 4.35(1H,dd, J = 10,4 Hz), 4.52(1H,d, J = 13 Hz), 6.73(1H,dd, J = 7,5 Hz), 7.09-7.17(4H,m), 7.31(1H,dd, J = 7,2 Hz), 8.15(1H,dd J = 5,2 Hz)

A 10.6 g portion of the previously obtained L-tartaric acid salt of S-mirtazapine was combined with a solution of 2.6 g of sodium hydroxide dissolved in 20 mL of water, and extraction was performed with tert-butyl methyl ether (20 mL × 2). Magnesium sulfate was used for dehydrating of the obtained organic layer, and the dehydrated organic layer was concentrated to obtain 6.10 g of S-mirtazapine as white crystals. The yield was 91% (based on mirtazapine (racemic mixture)).

### Reference Example 1

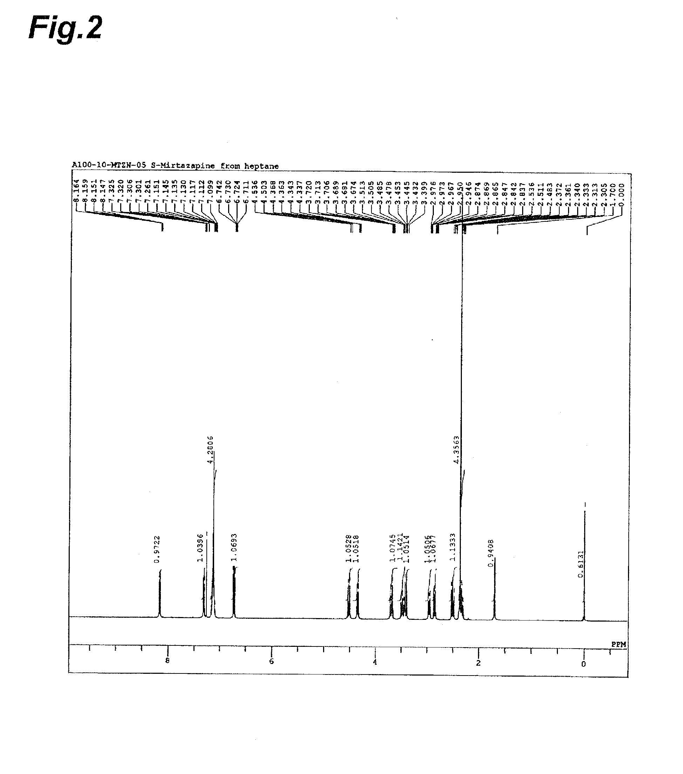

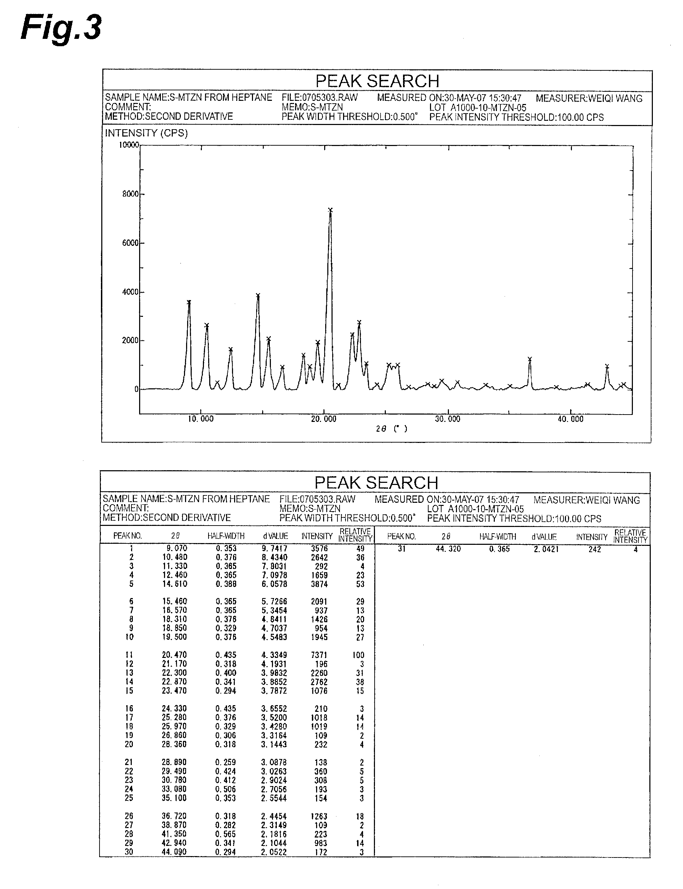

After combining 6.1 g of the S-mirtazapine obtained in Example 1 with 15 mL of heptane and dissolving the S-mirtazapine crystals at 50°C, the mixture was slowly cooled to 0 to 5°C, resulting in crystal deposition. The deposited crystals were obtained by filtration and then washed with 2 mL of heptane and dried to obtain 5.6 g of S-mirtazapine as white crystals. The crystal yield was 92%.
S-mirtazapine crystal property data:
Specific rotation [α]D23: 535° (c 0.1 methanol)
Melting point (3 measurements): 86.0°C, 85.8°C, 85.9°C
The 1H-NMR spectrum chart is shown in Fig. 2, and the XRD data are shown in Fig. 3.

### Reference Example 2

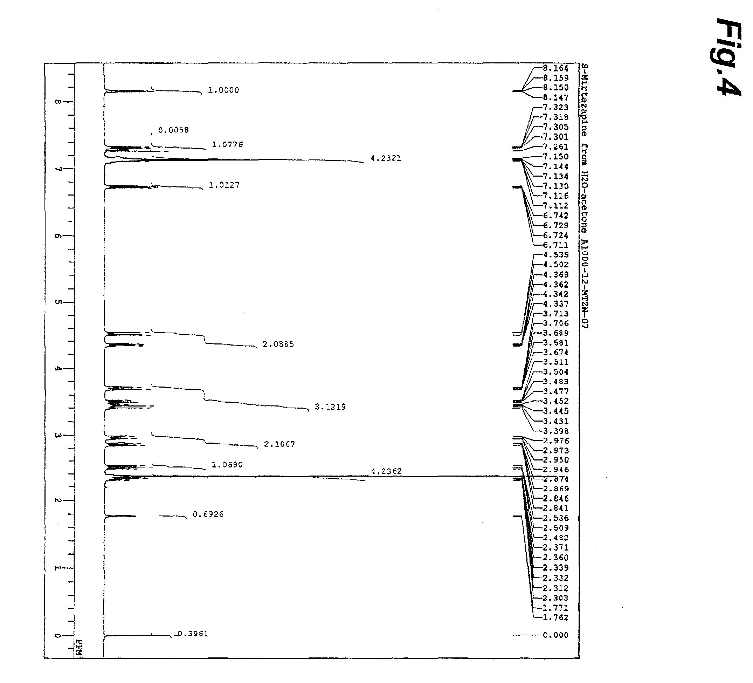

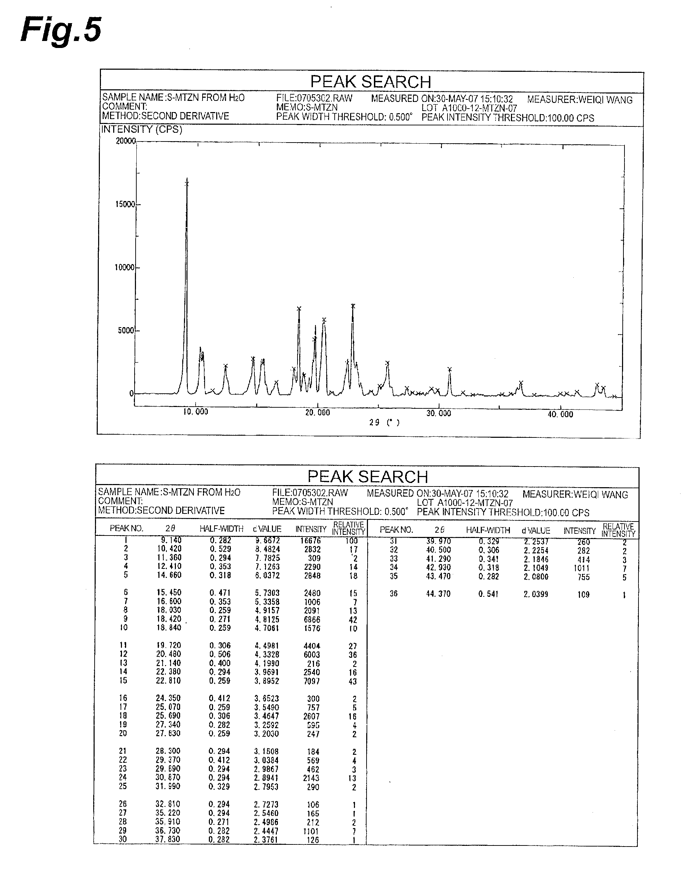

After dissolving 4.2 g of the S-mirtazapine obtained in Reference Example 1 in 4 mL of acetone, 10 mL of water was slowly added dropwise at 40°C, resulting in deposition of crystals. The obtained mixture was stirred at 40°C for 1 hour and then slowly cooled to 25°C. The deposited crystals were obtained by filtration and then washed with 2 mL of water and dried under reduced pressure at 25°C to obtain 4.0 g of S-mirtazapine as white crystals. The crystal yield was 95%.
S-mirtazapine crystal property data:
Melting point (3 measurements): 84.7°C, 85.2°C, 84.2°C
The 1H-NMR spectrum chart is shown in Fig. 4, and the XRD data are shown in Fig. 5.

### Example 2

After combining 15.0 g (56.5 mmol, racemic mixture) of mirtazapine, 8.65 g (57.6 mmol) of D-tartaric acid and 180 mL of methanol, the obtained mixture was heated to 50°C and stirred to obtain a homogeneous solution. Upon adding 18 mL of water dropwise thereto at the same temperature over a period of about 105 minutes, crystals were deposited. The obtained mixture was cooled to 25°C over a period of about 15 hours. The crystals were separated from the obtained mixture by filtration, washed with 25 mL of methanol-water (10: 1) and then dried under reduced pressure to obtain 12.8 g of crude crystals of the D-tartaric acid salt of R-mirtazapine as a white powder. The optical purity of the R-mirtazapine in the obtained crude crystals was 97.4% ee.
After mixing 12.8 g of the crude crystals with 60 mL of methanol, the mixture was heated to 50°C and mixed, causing complete dissolution of the crystals. Upon adding 6 mL of water thereto and mixing, crystals were deposited. The obtained mixture was cooled to 25°C over a period of about 21 hours. The crystals were separated from the obtained mixture by filtration, washed with 5 mL of methanol-water (10:1) and then dried under reduced pressure to obtain 10.7 g of crude crystals of the D-tartaric acid salt of R-mirtazapine as a white powder. The optical purity of the R-mirtazapine in the obtained crystals was 100% ee. The water content of the crystals was 9.2 wt% as measured by the Karl Fischer method.

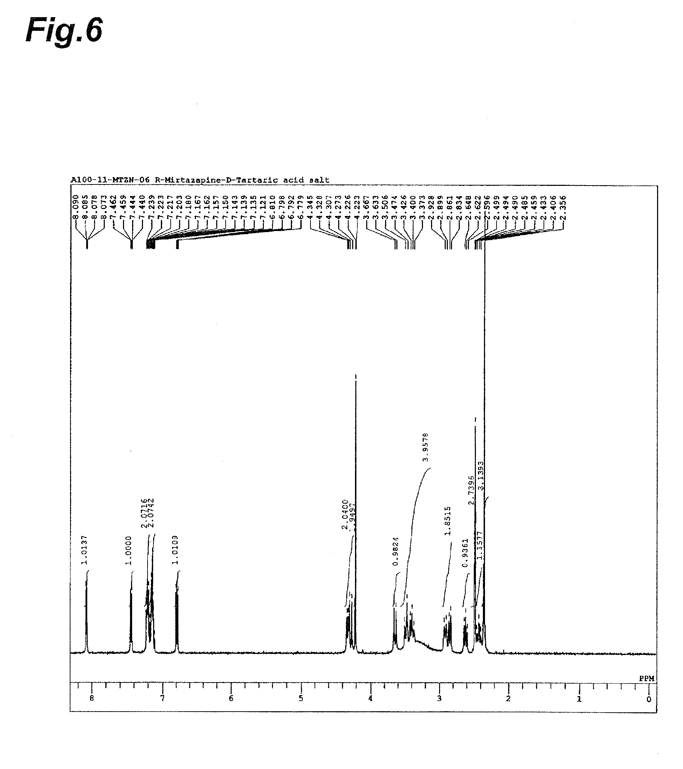

Fig. 6 shows the 1H-NMR spectrum chart for the D-tartaric acid salt of R-mirtazapine.

### Industrial Applicability

Optically active mirtazapine is useful as a therapeutic agent for sleep disorders, for example (see International Patent Publication No. 2006/51111), and according to the invention the compound can be efficiently and inexpensively produced.

## Claims

1. A process for production of optically active mirtazapine wherein an RS mixture of mirtazapine is optically resolved with optically active tartaric acid in the presence of a solvent.

2. A process for production of optically active mirtazapine, **characterized by** making an RS mixture of mirtazapine contact with optically active tartaric acid in the presence of a solvent, and preferentially crystallizing the optically active mirtazapine salt.

3. The process according to claim 2, wherein the obtained optically active mirtazapine salt is further made to contact with a base.

4. The process according to any one of claims 1 to 3, wherein the solvent is a mixed solvent consisting of a water-soluble organic solvent and water.

5. The process according to claim 4, wherein the water-soluble organic solvent is a C1-3 alcohol solvent.

6. The process according to any one of claims 1 to 5, wherein the optically active tartaric acid is L-tartaric acid and the obtained optically active mirtazapine is S-mirtazapine.

7. A salt consisting of optically active tartaric acid and optically active mirtazapine.

8. A salt consisting of L-tartaric acid and S-mirtazapine.

9. A salt consisting of D-tartaric acid and R-mirtazapine.
